# EUROPÄISCHE PATENTSCHRIFT

(11) **EP 3 180 053 B1**
(45) Veröffentlichungstag und Bekanntmachung des Hinweises auf die Patenterteilung: **11.11.2020**
(21) Anmeldenummer: 14755796.1
(22) Anmeldetag: 14.08.2014
(51) Int. Cl.: A61M 5/178

(54) **ABGABEVORRICHTUNG MIT WÄHLBAREN DURCHFLUSSENKANÄLEN UND DICHTUNG**
DELIVERY DEVICE HAVING SELECTABLE FLOW PASSAGES AND SEALING ELEMENT
DISPOSITIF DE DISTRIBUTION AYANT SÉLECTIONNABLE PASSAGE DU FLUIDE ET ÉLÉMENT D'ÉTANCHÉITÉ

(43) Veröffentlichungstag der Anmeldung: 21.06.2017
(73) Patentinhaber: Weibel CDS AG, 9104 Waldstatt (CH)
(72) Erfinder: WEIBEL, Ludwig Daniel, CH-9104 Waldstatt (CH); EGLOFF, Christoph, 8224 Löhningen (CH)
(74) Vertreter: SHL Medical
(86) Internationale Anmeldenummer: PCT/EP2014/067435
(87) Internationale Veröffentlichungsnummer: WO 2016/023590

(56) Entgegenhaltungen:
- EP-A1- 2 744 468
- WO-A2-2007/041512
- US-A- 2 261 213

## Beschreibung

Die vorliegende Erfindung betrifft eine Injektionsspritze zur Abgabe eines Fluids, insbesondere zur medizinischen Anwendung. Die Erfindung ist in Anspruch 1 definiert.

Es ist weitgehend bekannt, Spritzen zur Injektion von flüssigen Formulierungen pharmazeutischer Wirkstoffe an Patienten anzuwenden. Allerdings werden Spritzen im klinischen Umfeld beispielsweise auch zur Übertragung von Flüssigkeiten zwischen zwei Behältern oder von einem Behälter zu einem Katheter verwendet. Aus Gründen der Hygiene handelt es sich hierbei heutzutage meist um Einwegspritzen, die vor Gebrauch mit der abzugebenden Flüssigkeit befüllt werden müssen. Dabei ist zur Wahrung der Sterilität höchste Sorgfalt zu beachten, was insbesondere bei der Selbstabgabe von Medikamenten durch einen Patienten schwierig zu gewährleisten ist.

Um dieses Problem zu umgehen, sind vorgefüllte Spritzen bekannt, bei welchen eine Medikation bereits in das Abgabevolumen der Spritze vorgefüllt ist. Derartige Spritzen haben allerdings gewisse Nachteile hinsichtlich ihrer Lagerung. So muss beispielsweise eine Kolbenstange, mit welcher ein Kolben in der Spritze verschiebbar ist, weitgehend vollständig ausgefahren sein. Die Lagerung der Spritze in befülltem Zustand ist damit vergleichsweise platzintensiv und es müssen Vorkehrungen getroffen werden, dass die weit überstehende Kolbenstange nicht unbeabsichtigt betätigt wird.

Des Weiteren ist die Lagerung von pharmazeutischen Wirkstoffen in Lösung oft problematisch, in vielen Fällen sogar nicht praktisch umsetzbar. Probleme bestehen dabei neben der möglichen Zersetzlichkeit eines Wirkstoffes in Lösung in unerwünschten Wechselwirkungen zwischen den Materialien der Spritze und der flüssigen Formulierung. So stellt insbesondere der Kontakt der Flüssigkeit mit Dichtungsmaterialien und Schmierstoffen, welche erforderlich sind, damit sich der Kolben auch nach längerer Lagerdauer im Spritzenkörper problemlos verschieben lässt, ein Problem dar. Eine Kontamination der in der Spritze enthaltenen Lösung kann zu einer Überschreitung der regulatorisch festgelegten Grenzwerte oder im schlimmsten Fall sogar zu einer zumindest teilweisen Zersetzung des pharmazeutischen Wirkstoffes führen.

Insbesondere bei Wirkstoffen, die in Lösung nicht lagerfähig sind, ist es erforderlich, verschiedene Substanzen erst kurz vor der Verabreichung an einen Patienten miteinander zu mischen bzw. eine Lösung herzustellen. Dabei kann es sich beispielsweise um die Rekonstitution eines Lyophilisates handeln. Allerdings ist auch das Mischen zweier Flüssigkeiten denkbar. Hierzu müssen die verschiedenen Komponenten in getrennten Behältnissen aufbewahrt und unter sterilen Bedingungen vereint werden, bevor sie in eine Abgabevorrichtung, zum Beispiel eine Injektionsspritze, transferiert werden können.

Es besteht daher ein Bedarf an Abgabevorrichtungen, welche sich für eine Befüllung aus Behältern wie zum beispielsweise Glasphiolen unter möglichst sterilen Bedingungen eignen. Derartige Vorrichtungen sind beispielsweise in der EP 0 814 866 B1 oder in der EP 1 755 520 B1 beschrieben. Beide Dokumente beziehen sich auf Abgabevorrichtungen für flüssige Pharmazeutika, welche über Adapter mit Phiolen koppelbar sind. Über eine Durchflusssteuervorrichtung, welche im Wesentlichen die Funktion eines Dreiwegeventils erfüllt, können die besagten Phiolen jeweils bei Bedarf eine Fluidkommunikation mit einem Aufnahmeraum zur Aufnahme eines Fluids innerhalb der Abgabevorrichtung eingehen. Der Aufnahmeraum kann ferner mit einem Auslass, welcher beispielsweise in eine weitere Phiole oder eine Injektionsnadel mündet, verbunden werden. Durch die besagten Vorrichtungen ist eine sichere Verabreichung einer flüssigen Formulierung an einem Patienten, fallweise auch mit Rekonstitution eines Lyophilisates, möglich. WO 2007/041512 beschreibt eine Durchflusssteuervorrichtung als Teil eines Infusionssystems.

Ein Nachteil der beschriebenen Systeme besteht allerdings darin, dass insbesondere die Durchflusssteuervorrichtung einen verhältnismässig komplexen Aufbau hat, was deren Produktion, insbesondere in hohen Stückzahlen, beispielsweise durch Spritzguss, schwierig macht. Ein weiteres Problem stellt die Dichtigkeit der Durchflusssteuervorrichtung dar. Um diese in einem gewünschten Ausmass zu erreichen, müssen die fluidführenden Teile in einer hohen Präzision hergestellt sein. Ferner wird insbesondere für die Durchflusssteuervorrichtung in diesem Zusammenhang eine konische Geometrie verwendet, was wiederum den Einsatz von Schmierstoffen erforderlich macht, um ein Festsitzen der konischen Teile zu verhindern. Wie bereits erwähnt, ist die Verwendung von Schmierstoffen bei derartigen Vorrichtungen allerdings problematisch, da sich derartige Schmierzusammensetzungen über eine längere Lagerdauer nicht nur zersetzen können, sondern auch eine Kontamination der Formulierung durch das Schmiermittel möglich ist. Des Weiteren ist die erzielbare Dichtigkeit ein Problem, wenn es bei der Medikamentenabgabe innerhalb der Vorrichtung zu verhältnismässig hohen Drücken kommt. Dies kann beispielsweise bei der subkutanen Abgabe einer flüssigen Formulierung an einem Patienten der Fall sein, wo es wünschenswert ist, eine möglichst dünne Spritzennadel zu verwenden, um die Abgabe für den Patienten möglichst schmerzfrei zu gestalten.

Es ist daher die Aufgabe der Erfindung, die Nachteile im Stand der Technik zu überwinden.

Insbesondere ist es eine Aufgabe der Erfindung, eine einfach und sicher handhabbare Abgabevorrichtung zur Abgabe eines Fluids, bevorzugt zum einmaligen Gebrauch, bereitzustellen. Die Abgabevorrichtung soll zudem vielseitig anwendbar sein und eine einfache Konstruktion aufweisen. Sie soll zur Produktion in hohen Stückzahlen, insbesondere durch Spritzguss, geeignet sein. Ferner soll sie auch bei verhältnismässig hohen Flüssigkeitsdrücken eine gute Dichtigkeit gewährleisten.

Diese Aufgaben werden durch eine Injektionsspritze gelöst, welche die Merkmale in Anspruch 1 aufweist. Die besagte Injektionsspritze umfasst einerseits einen Grundkörper mit einer Längsachse (A), welcher einen vorzugsweise hohlzylindrischen Aufnahmeraum zur Aufnahme des Fluids und einen mit dem Aufnahmeraum in Verbindung stehenden Anschlusskanal aufweist. Der Anschlusskanal mündet dabei an einer Ventilfläche des Grundkörpers. Andererseits umfasst die Injektionsspritze ein Durchflusssteuerelement, welches beweglich am Grundkörper angeordnet ist. Dieses Durchflusssteuerelement weist einen oder mehrere Durchflusskanäle auf. Diese Durchflusskanäle münden an einer Ventilfläche des Durchflusssteuerelementes.

Die Ventilflächen des Grundkörpers und des Durchflusssteuerelements .können parallel zueinander angeordnet sein. Unter dem Begriff parallel wird dabei in Bezug auf Flächen verstanden, dass eine erste Fläche zu einer zweiten Fläche parallel ist, wenn jeder Punkt auf der ersten Fläche den Gleichen Abstand zu der zweiten Fläche hat. Der Abstand zwischen einem Punkt und einer Fläche bezieht sich dabei auf die Länge der Strecke die durch den Punkt und den zu ihm nächstliegenden Punkt der Fläche begrenzt wird.

Der Anschlusskanal des Grundkörpers und die Durchflusskanäle des Durchflusssteuerelements stehen direkt oder indirekt miteinander in Fluidverbindung oder sind durch Relativbewegung von Grundkörper und Durchflusssteuerelement miteinander in Fluidverbindung bringbar.

Die Erfindung zeichnet sich dadurch aus, dass zwischen der Ventilfläche des Grundkörpers und der Ventilfläche des Durchflusssteuerelementes ein Dichtungselement angeordnet ist.

Dieser Aufbau ermöglicht es, die gewünschte Ventilwirkung in einfacher Weise und verbesserter Zuverlässigkeit zu erzielen. Durch das Vorhandensein eines Dichtungselementes zwischen den Ventilflächen des Grundkörpers und des Durchflusssteuerelementes wird eine wesentlich bessere Dichtigkeit der Injektionsspritze erreicht. Dies ist insbesondere beim Auftreten von verhältnismässig hohen Drücken im Inneren der Injektionsspritze vorteilhaft. Ferner erlaubt das Vorhandensein eines Dichtungselementes, auf den Einsatz von Schmier- und/oder Dichtungsfetten zu verzichten. Dadurch wird eine deutlich verbesserte Lagerbeständigkeit der Injektionsspritze über längere Zeiträume ermöglicht. Auch ist damit eine Kontamination der die Injektionsspritze durchströmenden Flüssigkeit durch derartige Mittel ausgeschlossen. Ebenfalls wird ein Festsitzen von zueinander beweglichen Teilen selbst nach längerer Lagerung vermieden.

Bei der erfindungsgemässen Injektionsspritze bildet die Ventilfläche am Grundkörper bevorzugt eine Stirnseite des Grundkörpers. Dieser Aufbau ist besonders vorteilhaft, da eine derartige Injektionsspritze ohne gebogene Fluidkanäle, das heisst im Wesentlichen gerade Fluidkanäle, auskommen kann. Dies führt zu einer signifikanten Erleichterung einer maschinellen Fertigung der Injektionsspritze in hoher Stückzahl, insbesondere durch Spritzguss. Ferner kann durch das stirnseitige Aufliegen der beiden Ventilflächen eine konische Geometrie des Ventils vermieden werden, was ein Festsitzen der relativ zu einander beweglichen Teile nahezu verunmöglicht.

Die Ventilflächen können dabei Ebenen sein, wobei die Längsachse (A) des Grundkörpers vorzugsweise senkrecht zu diesen Ebenen verläuft. Dies stellt eine weitere Vereinfachung des Ventilaufbaus dar. Zudem können durch den in diesem Fall planaren Ventilaufbau Dichtungselemente verwendet werden, die im Wesentlichen aus flachen Scheiben eines elastischen Materials bestehen. Solche Dichtungselemente sind weit verbreitet und in vielfältigen Varianten erhältlich. Dies eröffnet einerseits einen grossen Spielraum bei der Auslegung der erfindungsgemässen Injektionsspritze, da die Materialzusammensetzung des Dichtungselements an das abzugebende Präparat angepasst werden kann. Ferner werden dadurch auch die Fertigungskosten gesenkt.

Vorzugsweise hat das Dichtungselement eine Ausdehnung in Richtung der Längsachse (A) des Grundkörpers von maximal 10 mm. Bevorzugterweise beträgt diese Ausdehnung zwischen 3 mm und 1 mm. Das Dichtungselement kann aus einem Elastomer, insbesondere aus einem thermoplastischen Elastomer, Naturkautschuk oder einem Synthesekautschuk beschaffen sein. Als Synthesekautschuke sind unter anderem Ethylen-Propylen-Kopolymere, Butylkautschuke, Polyether-Amide, Epoxid-Kautschuke, Urethan-Kautschuke, Silikon-Kautschuke oder Polysulfid-Kautschuke geeignet.

Der Grundkörper kann vorteilhafterweise ein unmittelbar an den Aufnahmeraum angrenzendes, vorzugsweise rotationssymmetrisches, bevorzugterweise zylindrisches oder kegelstumpfförmiges, Kopfstück aufweisen. Durch diese Ausgestaltung des Grundkörpers kann das Durchflusssteuerelement als Kappe mit einer Aufnahme ausgeführt werden, die auf das Kopfstück aufgesetzt wird. Dadurch wird einerseits eine sehr einfache und effiziente Montage der Injektionsspritze ermöglicht. Andererseits ist auch ein modularer Aufbau der Injektionsspritze realisierbar, bei welchem je nach Verwendungszweck verschiedene Grundkörper mit verschiedenen Durchflusssteuerelementen kombiniert werden können.

Das Durchflusssteuerelement kann aus einer ersten Steuerstellung, in welcher eine Fluidverbindung zwischen dem Anschlusskanal des Grundkörpers und mindestens einem Durchflusskanal des Durchflusssteuerelementes unterbrochen ist, in zumindest eine weitere zweite Steuerstellung bewegbar sein. In dieser zweiten Steuerstellung kann eine Fluidverbindung zwischen dem Anschlusskanal und einem Durchflusskanal bestehen, welche in der ersten Steuerstellung unterbrochen ist. In einer vorteilhaften Ausführungsform ist das Durchflusssteuerelement, vorzugsweise zusätzlich, von einer Steuerstellung in der zweiten Art in eine Steuerstellung der ersten Art bewegbar. Dadurch können je nach Verwendungszweck der Injektionsspritze verschiedene Fluidwege je nach Bedarf geöffnet oder geschlossen werden.

Allerdings kann das Durchflusssteuerelement auch mindestens einen Durchflusskanal aufweisen, der unabhängig von der Steuerstellung des Durchflusssteuerelementes mit dem Anschlusskanal des Grundkörpers in Fluidverbindung steht. Dies vereinfacht den Aufbau und die Funktionsweise der Injektionsspritze, wenn für einen Durchflusskanal keine eigentliche Ventilwirkung erforderlich ist.

Das Durchflusssteuerelement kann mindestens einen Durchflusskanal aufweisen, der zumindest im Bereich seiner Ventilfläche parallel zur Längsachse (A) des Grundkörpers verläuft. Ferner kann auch der Anschlusskanal am Grundkörper im Wesentlichen parallel zu dessen Längsachse (A) des Grundkörpers verlaufen. Eine Injektionsspritze mit einer derartigen Anordnung von Anschlusskanal und Durchflusskanälen lässt sich besonders leicht und kostengünstig herstellen.

Bevorzugt kann das Durchflusssteuerelement um eine, zur Längsachse (A) des Grundkörpers im Wesentlichen parallele, Drehachse drehbar am Grundkörper angeordnet und das Durchflusssteuerelement durch Drehung zwischen mehreren Steuerstellungen beweglich sein. Eine relative Rotationsbewegung zwischen Grundkörper und Durchflusssteuerelement ermöglicht es, die gewünschte Ventilwirkung möglichst einfach und platzsparend zu realisieren. Allerdings wäre prinzipiell auch eine Translatorische Relativbewegung von Grundkörper und Durchflusssteuerelement, beispielsweise durch Verschieben des Durchflusssteuerelements in einer Richtung senkrecht zur Längsachse (A) des Grundkörpers, denkbar.

Der Anschlusskanal am Grundkörper kann bezüglich der Längsachse (A) des Grundkörpers exzentrisch oder auch koaxial angeordnet sein. Dadurch sind insbesondere bei einer rotationsbeweglichen Ausführung von Grundkörper und Durchflusssteuerelement verschiedenste Ventilkonfigurationen denkbar.

Allerdings kann auch zumindest ein Durchflusskanal am Durchflusssteuerelement bezüglich der Längsachse (A) des Grundkörpers exzentrisch und/oder auch koaxial angeordnet sein. Auch dadurch wird ein noch breiteres Spektrum von Ventilkonfigurationen ermöglicht.

Ein relativer Bewegungsbereich vom Grundkörper und Durchflusssteuerelement kann durch eine Begrenzungsvorrichtung derart begrenzt sein, dass eine Bewegung nur von einer ersten bis in eine zweite Steuerstellung möglich ist. Auf diese Weise wird sichergestellt, dass der Benutzer das Durchflusssteuerelement nicht zu weit, das heisst über eine zweite Steuerstellung hinaus, bewegen kann.

Zusätzlich zur Begrenzungsvorrichtung kann an einer erfindungsgemässen Injektionsspritze vorteilhafterweise auch eine Sperrvorrichtung vorhanden sein, mit welcher die relative Bewegungsrichtung, insbesondere eine Drehrichtung, des Durchflusssteuerelementes gegenüber dem Grundkörper vorgegeben ist. Insbesondere kann eine Bewegungsrichtung von einer ersten Steuerstellung in eine zweite Steuerstellung vorgegeben sein. Auf diese Weise wird sichergestellt, dass ein Benutzer das Durchflusssteuerelement, beispielsweise nach einem Füllvorgang, nicht in eine falsche Richtung bewegen, insbesondere Drehen, kann. Die Sperrvorrichtung kann dabei nach Art einer Knarre ausgebildet sein und zum Beispiel Sperrklinken am Verschlusselement umfassen, welche in eine entsprechende Zahnung am Grundkörper eingreifen können, oder umgekehrt.

Durch das Vorhandensein einer Sperrvorrichtung in Kombination mit einer Begrenzungsvorrichtung ist zudem sichergestellt, dass das Durchflusssteuerelement beispielsweise in einer Abgabestellung arretiert ist, wenn diese einmal eingenommen wurde. Das Durchflusssteuerelement kann somit nicht mehr in eine Füllstellung gebracht werden, womit eine mehrmalige Benutzung der Injektionsspritze verhindert wird.

Bevorzugt weist der Grundkörper auf seiner Ventilfläche mindestens ein Element zum Verhindern einer relativen Rotation von Grundkörper und Dichtungselement auf. Dadurch kann vermieden werden, dass sich der Grundkörper und das Dichtungselement relativ zueinander verdrehen, was zu einem Blockieren des Anschlusskanals führen kann. Das Element zum Verhindern einer relativen Rotation von Grundkörper und Dichtungselement kann dabei beispielsweise als Mündungsstutzen des Anschlusskanals ausgestaltet sein. Bei einer derartigen Ausführung muss die Ventilfläche des Grundkörpers bis auf die Mündung des Anschlusskanals nicht verändert werden, beispielsweise durch eine zusätzliche Verdrehsicherung. Ferner ist durch diese Anordnung sichergestellt, dass das Dichtungselement insbesondere im Mündungsbereich des Anschlusskanals korrekt auf der Ventilfläche des Grundkörpers liegt.

Zwischen dem Anschlusskanal des Grundkörpers und mindestens einem Durchflusskanal des Durchflusssteuerelements kann über das Dichtungselement eine indirekte Fluidverbindung herstellbar sein. So kann zum Beispiel über das Dichtungselement eine indirekte Fluidverbindung zwischen einem mit der Längsachse (A) des Grundkörpers koaxial angeordneten Anschlusskanal und mindestens einem bezüglich der Längsachse (A) des Grundkörpers exzentrisch angeordneten Durchflusskanal am Durchflusssteuerelement herstellbar sein. Ferner besteht die Möglichkeit, dass neben zumindest einem bezüglich der Längsachse (A) des Grundkörpers exzentrisch angeordneten Durchflusskanal am Durchflusssteuerelement ein weiterer Durchflusskanal vorhanden ist, welcher unabhängig von der Steuerstellung des Durchflusssteuerelementes mit dem Anschlusskanal des Grundkörpers verbunden ist.

Optional kann der weitere Durchflusskanal, welcher unabhängig von der Steuerstellung des Durchflusselementes mit dem Anschlusskanal des Grundkörpers verbunden ist, koaxial mit der Längsachse (A) des Grundkörpers angeordnet sein.

Allerdings ist es auch möglich, dass über das Dichtungselement eine indirekte Fluidverbindung zwischen einem bezüglich der Längsachse (A) des Grundkörpers exzentrisch angeordneten Anschlusskanal und einen bezüglich der Längsachse (A) des Grundkörpers koaxial oder exzentrisch angeordneten Durchflusskanal am Durchflusssteuerelement herstellbar ist.

Dies verdeutlicht die hohe Flexibilität einer erfindungsgemässen Injektionsspritze bezüglich der realisierbaren Ventilkonfigurationen.

In einer bevorzugten Ausführungsform der vorliegenden Erfindung ist das Dichtungselement durch Bewegen des Durchflusssteuerelementes von einer ersten Steuerstellung in eine zweite Steuerstellung komprimierbar. Eine solche Kompression kann insbesondere dadurch erfolgen, dass der Abstand zwischen der Ventilfläche am Grundkörper und der Ventilfläche am Durchflusssteuerelement durch bewegen des Durchflusssteuerelementes verringert wird. Dies kann beispielsweise durch eine schiefe Ebene, insbesondere durch einen Gewindegang, am Grundkörper erfolgen, wodurch das Durchflusssteuerelement bei einer Drehbewegung gegen den Grundkörper verschoben wird. Eine Kompression des Dichtungselementes ist insbesondere wünschenswert, um die Dichtwirkung des Dichtungselements zu gewährleisten, bzw. zu erhöhen. Dies kann insbesondere bei längerer Lagerung einer erfindungsgemässen Injektionsspritze vor deren Verwendung von Bedeutung sein.

Eine Kanüle, insbesondere eine Injektionsnadel oder eine Spülkanüle, ist derart am Durchflusssteuerelement angeordnet oder anordenbar, dass eine fluiddichte Fluidverbindung mit einem Durchflusskanal besteht, wobei die Kanüle fest in den Durchflusskanal eingesetzt und insbesondere parallel zur Längsachse (A) des Grundkörpers angeordnet ist.

Der Begriff Kanüle bezeichnet in diesem Zusammenhang eine Hohlnadel mit einem Innenraum, welcher zur Führung eines Fluids ausgebildet ist. Eine Kanüle bezieht sich vorliegend insbesondere eine Injektionsnadel zum subkutanen, intravaskulären oder intramuskulären Einbringen eines Medikamentes in den menschlichen oder tierischen Körper (zu injizieren) oder zur Entnahme (zu punktieren) von Körperflüssigkeiten. Ebenso sind auch sogenannte Spülkanülen umfasst, bei welcher das Fluid zum Beispiel in bestehende Öffnungen (Körperhöhlen) gespritzt werden kann, ohne ein Gewebe zu verletzen.

Die Kanüle ist direkt in den Durchflusskanal eingesetzt, beispielsweise eingeklebt. Bevorzugterweise ist das Durchflusssteuerelement als Spritzgussteil ausgebildet. In diesem Fall kann die Kanüle beim Spritzen des Durchflusssteuerelements direkt umspritzt werden. Für eine sichere Befestigung kann sich die Kanüle Längs des gesamten Durchflusskanals bis zur Ventilfläche erstrecken.

Das Durchflusssteuerelement kann Kopplungsmittel zum Ankoppeln der Injektionsspritze an eine weitere Vorrichtung umfassen. Die weitere Vorrichtung kann beispielsweise eine Füllvorrichtung, eine Transfervorrichtung zum Transfer eines Fluids oder aber auch nur eine Schutzkappe zur Aufnahme einer Kanüle sein.

Ferner können an der Injektionsspritze Arretierungsmittel, insbesondere nach der Art eines Bajonetts, vorhanden sein, mit welchen die Injektionsspritze an komplementären Arretierungsmitteln einer weiteren Vorrichtung arretierbar ist. Bevorzugt sind die Arretierungsmittel am Grundkörper ausgebildet. Ein komplementärer Teil des bajonettartigen Verschlusses ist in diesem Fall entsprechend an der weiteren Vorrichtung ausgebildet. Bevorzugt ist die zum lösen des Arretierungsmittels erforderliche Drehung derart bemessen, dass bei entsprechender Drehung des Verschlusselements in Gegenrichtung ein Übergang von einer ersten Steuerstellung in eine zweite Steuerstellung erfolgt.

Mit Vorteil umfasst die weitere Vorrichtung eine Schutzkappe, insbesondere mit einem Aufnahmeraum zur Aufnahme einer Kanüle, welche einen Sitz für die Kopplungsmittel des Durchflusssteuerelementes aufweist. Auf diese Weise kann die Schutzkappe auf einfache Weise auf das Durchflusssteuerelement aufgesetzt, bzw. damit gekoppelt, werden.

Bevorzugt weist diese Schutzkappe einen Anschlussstutzen zum Anschluss einer Fluidquelle und ein Transferkanal zum Transfer eines Fluids vom Anschlussstutzen zu einer am Sitz ausgebildeten Mündungsöffnung auf. Auf diese Weise kann die Schutzkappe gleichzeitig die Funktion einer Transfervorrichtung erfüllen, welche zum Befüllen der Injektionsspritze, beispielsweise an einer Fluidquelle oder einer Phiole, anschliessbar ist. Die Mündungsöffnung ist vorzugsweise derart ausgebildet und am Sitz angeordnet, dass bei im Sitz angeordneten Kopplungsmitteln des Durchflusssteuerelements eine, insbesondere fluiddichte, vorzugsweise für Keime undurchlässige, Fluidverbindung zwischen der Mündungsöffnung am Sitz und dem Durchflusskanal des Durchflusssteuerelements besteht. Die Schutzkappe bzw. der Anschlussstutzen kann dabei derart ausgebildet sein, dass sie an bekannte Systeme zum Transfer eines Fluids anschliessbar ist.

Weitere Vorteile und Einzelmerkmale der Erfindung ergeben sich aus der nachfolgenden Beschreibung mehrerer Ausführungsbeispiele und aus den Figuren.

Es zeigen schematisch:
- Figur 1:: Einen Längsschnitt durch eine erfindungsgemässe Injektionsspritze, bei welcher das Durchflusssteuerelement sich in einer ersten Steuerposition befindet und darauf eine Schutzkappe aufgesetzt ist;
- Figur 2:: eine Teilvergrösserung des Längsschnitts durch die Injektionsspritze gemäss Figur 1;
- Figur 3:: einen Längsschnitt durch die Injektionsspritze gemäss Figur 1, wobei sich das Durchflusssteuerelement in einer zweiten Steuerposition befindet;
- Figur 4:: einen Längsschnitt durch ein alternatives Ausführungsbeispiel einer erfindungsgemässen Injektionsspritze, wobei sich das Durchflusssteuerelement in einer ersten Steuerposition befindet und darauf eine Schutzkappe aufgesetzt ist;
- Figur 5:: einen Längsschnitt durch die Injektionsspritze gemäss Figur 4, wobei das Durchflusssteuerelement sich in einer zweiten Steuerstellung befindet;
- Figur 6:: einen Längsschnitt durch ein weiteres alternatives Ausführungsbeispiel einer erfindungsgemässen Injektionsspritze, wobei das Durchflusssteuerelement sich in einer ersten Steuerposition befindet und darauf eine Schutzkappe aufgesetzt ist;
- Figur 7:: einen Längsschnitt durch die Injektionsspritze gemäss Figur 6, wobei das Durchflusssteuerelement sich in einer zweiten Steuerstellung befindet;
- Figur 8:: einen Querschnitt durch die Injektionsspritze gemäss Figur 1;
- Figur 9:: einen Querschnitt durch die Injektionsspritze gemäss Figur 3;
- Figur 10:: einen Querschnitt durch die Injektionsspritze gemäss Figur 4;
- Figur 11:: einen Querschnitt durch die Injektionsspritze gemäss Figur5;
- Figur 12:: einen Querschnitt durch die Injektionsspritze gemäss Figur 6;
- Figur 13:: einen Querschnitt durch die Injektionsspritze gemäss Figur 7;
- Figur 14:: eine perspektivische Darstellung eines Grundkörpers einer erfindungsgemässen Injektionsspritze;
- Figur 15:: eine perspektivische Darstellung eines komplementären Durchflusssteuerelements zum Grundkörper gemäss Figur 14;
- Figur 16:: eine Aufsicht auf eine erfindungsgemässe Injektionsspritze mit den Komponenten gemäss den Figuren 14 und 15;
- Figur 17:: eine perspektivische Darstellung einer Schutzkappe für die Injektionsspritze gemäss Figur 16;
- Figur 18:: eine Aufsicht auf die Injektionsspritze gemäss Figur 16, auf welche eine Schutzkappe gemäss Figur 17 aufgesetzt ist;
- Figur 19:: ein vergrösserter Teilschnitt in Längsrichtung durch die Injektionsspritze mit Schutzkappe gemäss Figur 18.

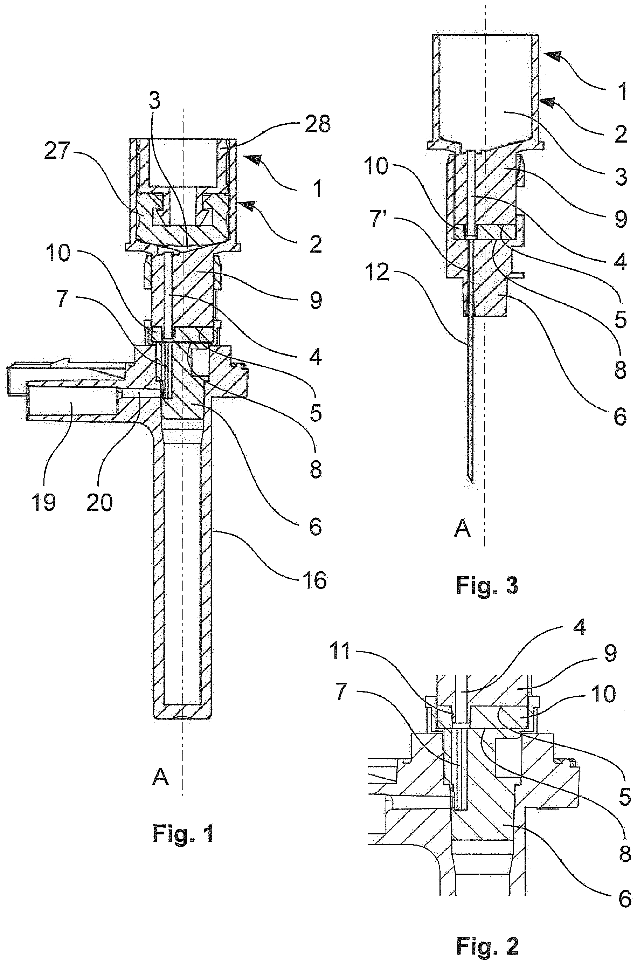

Figur 1 zeigt einen Längsschnitt durch eine erfindungsgemässe Injektionsspritze 1, bei welcher ein Durchflusssteuerelement 6 sich in einer ersten Steuerposition befindet und darauf eine Schutzkappe 16 aufgesetzt ist. Die Injektionsspritze 1 besteht einerseits aus einem Grundkörper 2 mit einer Längsachse A, welcher einen hohlzylindrischen Aufnahmeraum 3 zur Aufnahme eines Fluids aufweist.

Das Durchflusssteuerelement 6 weist zwei Durchflusskanäle 7 und 7' (in der Längsschnittdarstellung nur Durchflusskanal 7 sichtbar) auf. Der Anschlusskanal 4 am Grundkörper 2 und die Durchflusskanäle 7, 7' am Durchflusssteuerelement sind jeweils parallel zur Längsachse A des Grundkörpers 2 ausgerichtet, jedoch exzentrisch positioniert. Innerhalb des Aufnahmeraums 3 des Grundkörpers 2 ist ein Kolben 27 über eine zylindrische Kolbenstange 28 verschiebbar angeordnet. An den Aufnahmeraum 3 grenzt ein zylindrisch ausgebildetes Kopfstück 9 an, durch welches der Anschlusskanal 4 führt. Der Anschlusskanal 4 steht in Fluidverbindung mit dem Innenraum 3 zur Aufnahme des Fluids und mündet an der Ventilfläche 5 des Grundkörpers 2. Parallel zu der besagten Ventilfläche 5 verläuft eine Ventilfläche 8 des Durchflusssteuerelements, wobei zwischen den beiden planparallelen Ventilflächen 5 und 8 ein Dichtungselement 10 angeordnet ist.

Damit stehen der Anschlusskanal 4 und der Durchflusskanal 7 in Fluidverbindung. Der Durchflusskanal 7 im Durchflusssteuerelement 6 ist weiter, mit einem Transferkanal 20 an der Schutzkappe 16 verbunden, welcher in den Anschlussstutzen 19 mündet. Durch Betätigung der Kolbenstange 28 ist es daher möglich, mit dem Kolben 27 einen Unterdruck im Aufnahmeraum 3 zu erzeugen, wodurch eine Flüssigkeit vom Anschlussstutzen 19 über den Transferkanal 20, den Durchflusskanal 7 und den Anschlusskanal 4 in den Aufnahmeraum 3 aufgezogen werden kann.

In Figur 2 ist eine Teilvergrösserung eines Längsschnitts durch eine erfindungsgemässe Injektionsspritze gemäss Figur 1 gezeigt, wobei der Bereich um das Durchflusssteuerelement 6 detaillierter dargestellt ist. Es ist zu erkennen, dass zwischen der Ventilfläche 5 des Grundkörpers 2 und der Ventilfläche 8 des Durchflusssteuerelements 6 ein Dichtungselement 10 angeordnet ist. Um eine unerwünschte Relativrotation zwischen dem Grundkörper 2 und dem Dichtungselement 10 zu verhindern, ist am Kopfstück 9 des Grundkörpers 2 auf der Ventilfläche 5 ein Mündungsstutzen 11 angebracht, an welchem der Anschlusskanal 4 mündet. Bei einer Drehung des Durchflusssteuerelements 6 liegt das Dichtungselement 10 damit statisch auf der Ventilfläche 5 des Grundkörpers 2 auf. Ebenfalls ist in der besagten Abbildung die Fluidverbindung zwischen dem Anschlusskanal 4 und dem Durchflusskanal 7' mit dem Dichtungselement 10 detaillierter gezeigt.

Figur 3 zeigt die Injektionsspritze 1 gemäss Figur 1, wobei das Durchflusssteuerelement 6 durch Rotation um eine mit der Längsachse A des Grundkörpers 2 koaxiale Drehachse in eine zweite Steuerposition gebracht ist. Deutlich ist zu erkennen, dass eine Injektionskanüle 12, welche in einem Durchflusskanal 7' eingebettet ist, durch die Rotation innerhalb der Schnittebene liegt. Zudem steht der besagte Durchflusskanal 7' nun in Fluidkommunikation mit dem Anschlusskanal 4.

Durch Betätigen der Kolbenstange 28 und Verschieben des Kolbens 27 in Richtung des Anschlusskanals 4 kann der Inhalt des Aufnahmeraums 3 über den Anschlusskanal 4, den Durchflusskanal 7' und die Kanüle 12 aus der Injektionsspritze 1 abgegeben werden.

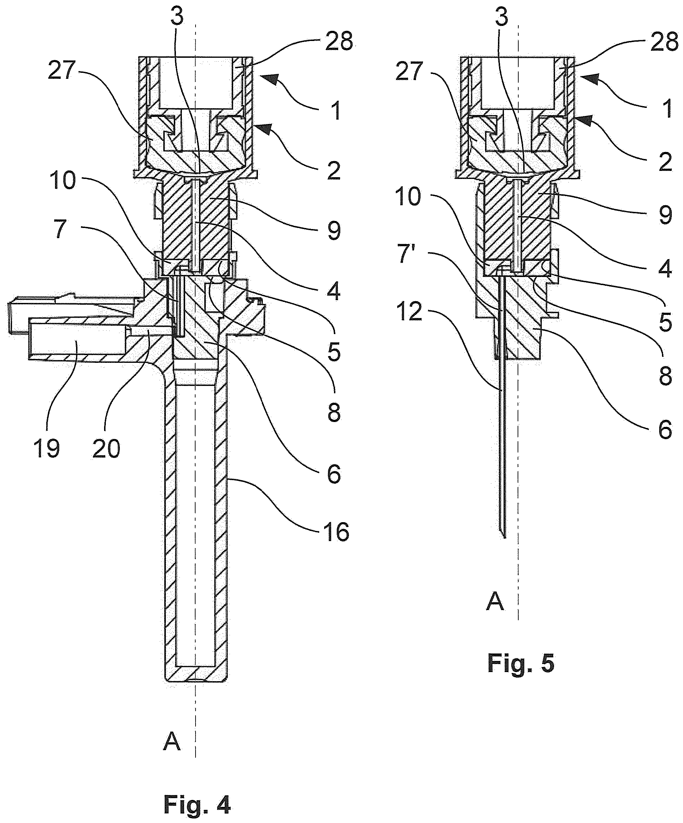

Die Figuren 4 und 5 zeigen ein alternatives Ausführungsbeispiel einer erfindungsgemässen Injektionsspritze 1, bei welchem der Anschlusskanal 4 am Grundkörper 2 koaxial mit dessen Längsachse A positioniert ist. Die Durchflusskanäle 7 und 7' im Durchflusssteuerelement 6 sind hingegen exzentrisch angeordnet. Um eine Fluidverbindung zwischen dem Anschlusskanal 4 am Grundkörper und den Durchflusskanälen 7 und 7' am Durchflusssteuerelement 6 herzustellen, ist das Dichtungselement 10 derart ausgestaltet, dass es einen indirekten Flüssigkeitspfad bildet. Figur 4 zeigt die Injektionsspritze 1 mit dem Durchflusssteuerelement 6 in einer ersten Steuerstellung, in welcher der Aufnahmeraum 3 des Grundkörpers 2 vom Anschlussstutzen 19 her über Transferkanal 20, den Durchlaufkanal 7 und den Anschlusskanal 4 befüllt werden kann. In Figur 5 ist dieselbe Injektionsspritze mit dem Durchflusssteuerelement in einer zweiten Steuerstellung zu sehen, in welcher der Anschlusskanal 4 mit dem Durchflusskanal 7' und der Injektionskanüle 12 derart verbunden ist, dass eine Abgabe des Fluids aus dem Aufnahmeraum 3 möglich ist.

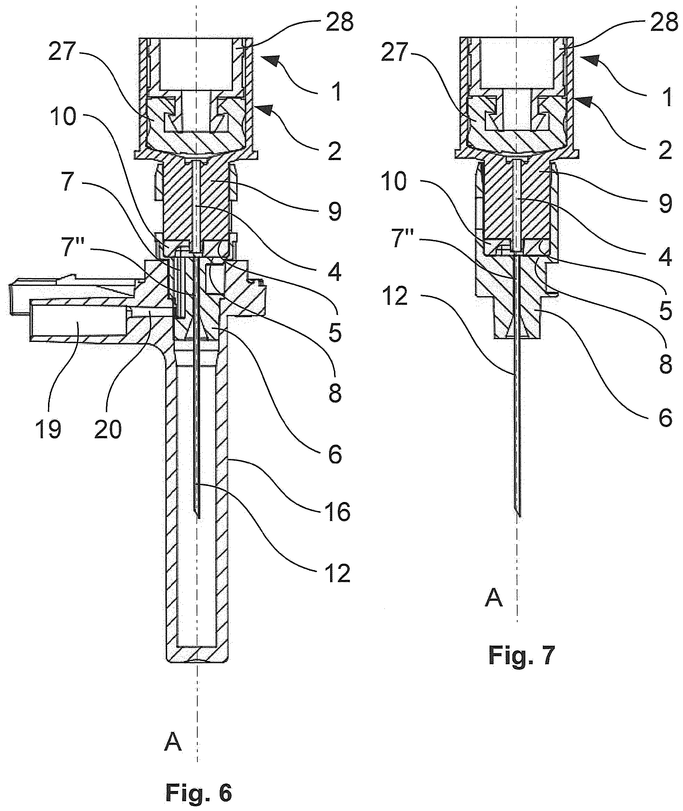

Die Figuren 6 und 7 zeigen ein weiteres Ausführungsbeispiel einer erfindungsgemässen Injektionsspritze, bei welcher der Anschlusskanal 4 am Grundkörper 2 koaxial mit dessen Längsachse A ausgeführt ist. Das Durchflusssteuerelement 6 weist hier hingegen zwei Durchflusskanäle 7 und 7" auf, welche zwar beide ebenfalls parallel zur Längsachse A des Grundkörpers 2 ausgerichtet sind, 7 allerdings exzentrisch und 7" koaxial angeordnet ist. Wie in Figur 6 zu erkennen ist, ist das Dichtungselement 10 derart ausgestaltet, dass es in einer ersten Steuerposition eine Fluidverbindung zwischen dem Anschlusskanal 4 am Grundkörper 2 und beiden Durchflusskanälen 7 und 7" am Durchflusssteuerelement 6 herstellt. In dieser Position ist ein Aufziehen von Flüssigkeit in den Aufnahmeraum 3 des Grundkörpers 2 möglich. Zwar ist die Kanüle 12 ebenfalls mit dem Aufnahmeraum 3 in Fluidkommunikation, da sie allerdings durch die Schutzkappe 16 indirekt verschlossen ist, stellt dies zum Befüllen der Injektionsspritze kein Problem dar. In Figur 7 ist dieselbe erfindungsgemässe Injektionsspritze mit dem Durchflusssteuerelement 6 in einer zweiten Steuerposition gezeigt. Es ist zu erkennen, dass nur noch der Durchflusskanal 7" mit dem Anschlusskanal 4 am Grundkörper 2 in Fluidkommunikation steht, wobei in diesem die Injektionskanüle 12 eingebettet ist. Die Injektionsspritze 1 ist damit bereit zur Abgabe einer Flüssigkeit.

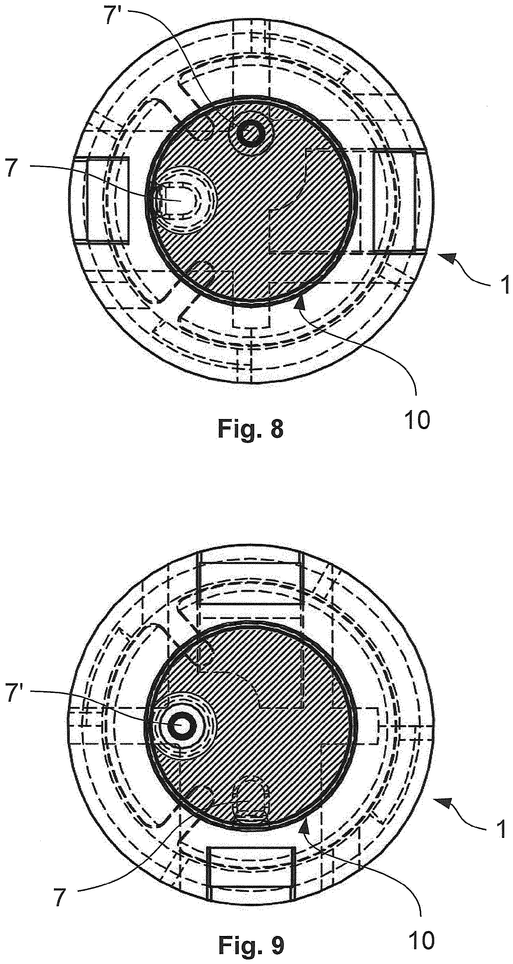

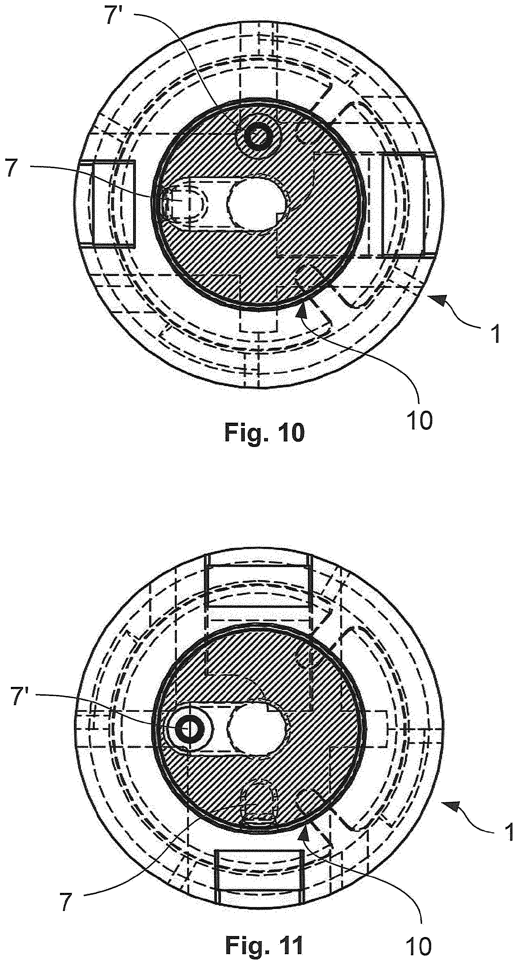

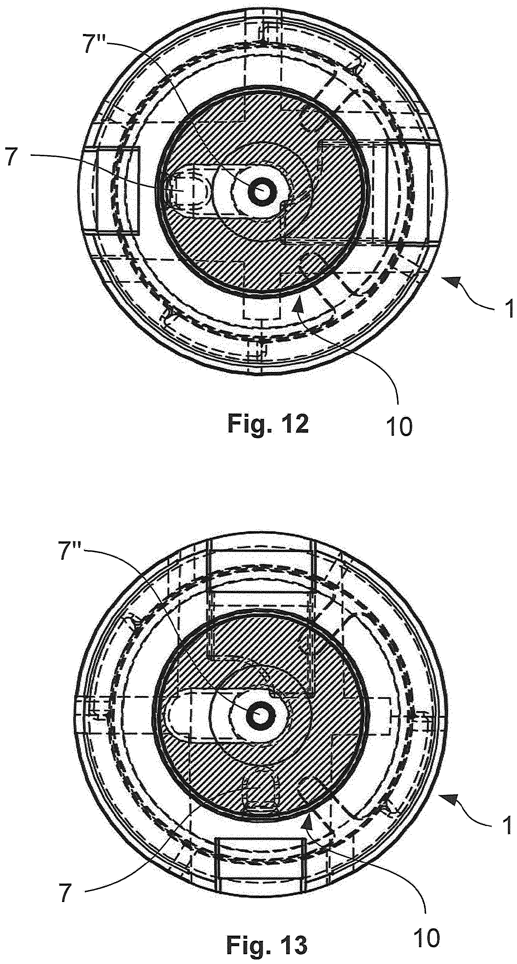

Die Figuren 8 bis 13 zeigen Querschnitte durch Injektionsspritzen 1 gemäss den Figuren 1, 3 und 4 bis 7. Zur besseren Übersichtlichkeit ist das Dichtungselement 10 jeweils schraffiert eingezeichnet. Deutlich ist zu erkennen, dass je nach Steuerposition des Durchflusssteuerelementes 6 über das Dichtungselement 10 unterschiedliche Fluidwege zwischen dem Anschlusskanal 4 (in diesen Figuren nicht sichtbar) und den Durchflusskanälen 7 und 7' geöffnet und/oder geschlossen werden. Der Durchflusskanal 7" ist hingegen unabhängig von der Steuerposition des Durchflusssteuerelementes 6 immer mit dem Anschlusskanal 4 verbunden (Figuren 12 und 13).

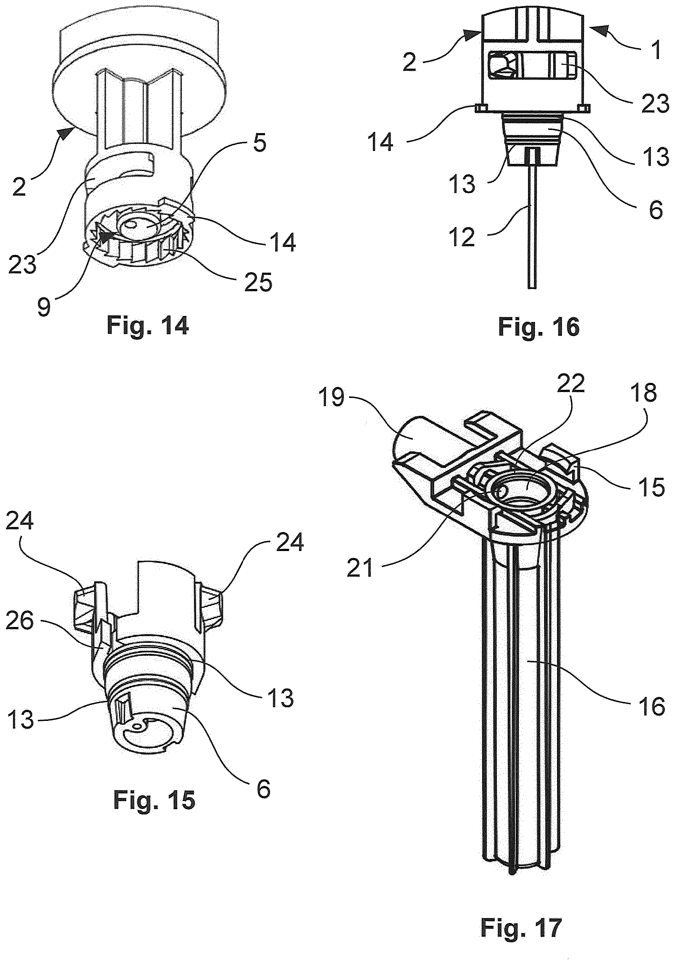

Figur 14 zeigt eine perspektivische Darstellung eines Grundkörpers 2 einer erfindungsgemässen Injektionsspritze 1. Deutlich ist das Kopfstück 9 mit der Ventilfläche 5 zu erkennen. Ebenfalls ist die gezeigte Ausführungsform mit einer Begrenzungsvorrichtung und einer Sperrvorrichtung versehen, wobei hier nur die am Grundkörper 2 angebrachten Teile (Nut 23 und Zahnung 25) zu sehen sind. Zudem sind am Grundkörper 2 Arretierungsmittel 14 in der Art eines Bajonettverschlusses angebracht.

In Figur 15 ist ein Durchflusssteuerelement 6 passend zum Grundkörper 2 gemäss Figur 14 gezeigt. Als Teil der Begrenzungsvorrichtung bzw. der Sperrvorrichtung sind die Eingriffelemente 24 bzw. die Sperrklinken 26 zu erkennen. Ferner sind am Durchflusssteuerelement 6 Kopplungsmittel 13 angebracht.

Figur 16 zeigt eine Aufsicht auf eine erfindungsgemässe Injektionsspritze mit einem Grundkörper gemäss Figur 14 und einem Durchflusssteuerelement gemäss Figur 15. Es ist zu erkennen wie die Eingriffelemente 24 in die Nuten 23 der Begrenzungsvorrichtung greifen, um die Rotation des Durchflusssteuerelementes 6 zu begrenzen.

Figur 17 zeigt eine perspektivische Darstellung einer Schutzkappe 16 für eine Injektionsspritze 1 gemäss Figur 16. Die besagte Schutzkappe 16 weist einen Sitz 18 für die am Durchflusssteuerelement 6 angebrachten Kopplungsmittel 13 sowie eigene Kopplungsmittel 22 auf. An diesem Sitz ist zudem eine Mündungsöffnung 21 ausgebildet, welche über den Transferkanal 20 mit dem Anschlussstutzen 19 verbunden ist. Ausserdem verfügt die gezeigte Schutzkappe 16 über Arretiermittel 15, die in der Art eines Bajonettverschlusses ausgebildet sind.

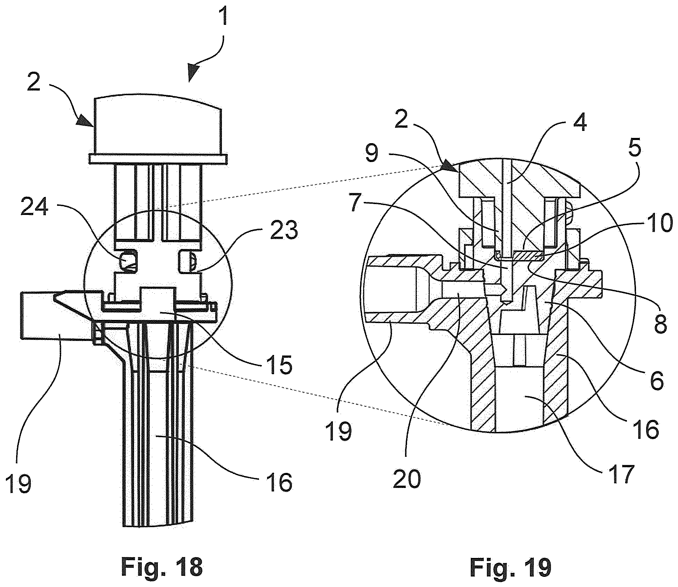

Figur 18 zeigt eine erfindungsgemässe Injektionsspritze 1 gemäss Figur 16, auf welche eine Schutzkappe 16 gemäss Figur 17 aufgesetzt ist. In Figur 19 ist ein Teilbereich von Figur 18 vergrössert als Längsschnitt gezeigt. In dieser detaillierten Darstellung ist der Fluidpfad zum Befüllen der Injektionsspritze 1 zu erkennen, welcher durch den Anschlussstutzen 19, den Transferkanal 20, den Durchflusskanal 7 und den Anschlusskanal 4 gebildet wird. Auch ist der Hohlraum 17 innerhalb der Schutzkappe 16 gezeigt.

## Patentansprüche

1. Injektionsspritze (1) zur Abgabe eines Fluids, insbesondere zur medizinischen Anwendung, umfassend
a) einen Grundkörper (2) mit einer Längsachse (A), welcher einen vorzugsweise hohlzylindrischen Aufnahmeraum (3) zur Aufnahme des Fluids und einen mit dem Aufnahmeraum (3) in Verbindung stehenden Anschlusskanal (4) aufweist, wobei der Anschlusskanal (4) an einer Ventilfläche (5) des Grundkörpers (2) mündet,
b) ein Durchflusssteuerelement (6), welches beweglich am Grundkörper (2) angeordnet ist und einen oder mehrere Durchflusskanäle (7, 7', 7") aufweist, wobei die Durchflusskanäle (7, 7', 7") an einer Ventilfläche (8) des Durchflusssteuerelementes (6) münden,
wobei der Anschlusskanal (4) des Grundkörpers (2) und die Durchflusskanäle (7, 7', 7") des Durchflusssteuerelements (6) direkt oder indirekt miteinander in Fluidverbindung stehen oder durch Relativbewegung von Grundkörper (2) und Durchflusssteuerelement (6) in Fluidverbindung bringbar sind, wobei zwischen der Ventilfläche (5) des Grundkörpers (2) und der Ventilfläche (8) des Durchflusssteuerelements (6) ein Dichtungselement (10) angeordnet ist, wobei eine Kanüle (12), insbesondere eine Injektionsnadel oder eine Spülkanüle, derart am Durchflusssteuerelement (6) angeordnet oder anordenbar ist, dass eine fluiddichte Fluidverbindung mit einem Durchflusskanal besteht (7', 7"), wobei die Kanüle (12) fest in den Durchflusskanal (7', 7") eingesetzt und insbesondere parallel zur Längsachse (A) des Grundkörpers (2) angeordnet ist.

2. Injektionsspritze (1) nach Anspruch 1, **dadurch gekennzeichnet, dass** der Anschlusskanal (4) am Grundkörper (2) im Wesentlichen parallel zu dessen Längsachse (A) des Grundkörpers verläuft.

3. Injektionsspritze (1) nach einem der Ansprüche 1 oder 2, **dadurch gekennzeichnet, dass** das Durchflusssteuerelement (6) um eine, zur Längsachse (A) des Grundkörpers (2) im Wesentlichen parallele, Drehachse drehbar am Grundkörper (2) angeordnet ist und das Durchflusssteuerelement (6) durch Drehung zwischen mehreren Steuerstellungen beweglich ist.

4. Injektionsspritze (1) nach Anspruch 2 in Verbindung mit Anspruch 3, **dadurch gekennzeichnet, dass** der Anschlusskanal (4) am Grundkörper (2) bezüglich der Längsachse (A) des Grundkörpers exzentrisch angeordnet ist.

5. Injektionsspritze (1) nach Anspruch 2 in Verbindung mit Anspruch 3, **dadurch gekennzeichnet, dass** der Anschlusskanal (4) am Grundkörper (2) koaxial mit der Längsachse (A) des Grundkörpers angeordnet ist.

6. Injektionsspritze (1) nach einem der Ansprüche 3 bis 5, **dadurch gekennzeichnet, dass** zumindest ein Durchflusskanal (7, 7') am Durchflusssteuerelement (6), bezüglich der Längsachse (A) des Grundkörpers (2) exzentrisch angeordnet ist.

7. Injektionsspritze (1) nach einem der Ansprüche 3 bis 6, **dadurch gekennzeichnet, dass** ein Durchflusskanal (7") am Durchflusssteuerelement (6) koaxial mit der Längsachse (A) des Grundkörpers (2) angeordnet ist.

8. Injektionsspritze (1) nach Anspruch 3, **dadurch gekennzeichnet, dass** der Grundkörper (2) auf seiner Ventilfläche (5) mindestens ein Element zum Verhindern einer relativen Rotation von Grundkörper (2) und Dichtungselement (6) aufweist.

9. Injektionsspritze (1) nach Anspruch 8, **dadurch gekennzeichnet, dass** das Element zum Verhindern einer relativen Rotation von Grundkörper (2) und Dichtungselement (10) als Mündungsstutzen (11) des Anschlusskanals (4) ausgestaltet ist.

10. Injektionsspritze (1) nach einem der Ansprüche 1 bis 9, **dadurch gekennzeichnet, dass** zwischen dem Anschlusskanal (4) des Grundkörpers (2) und mindestens einem Durchflusskanal (7, 7', 7") des Durchflusssteuerelementes (6) über das Dichtungselement (10) eine indirekte Fluidverbindung herstellbar ist.

11. Injektionsspritze (1) nach Anspruch 10 in Verbindung mit den Ansprüchen 4 und 5, **dadurch gekennzeichnet, dass** über das Dichtungselement (10) eine indirekte Fluidverbindung zwischen einem mit der Längsachse (A) des Grundkörpers (2) koaxial angeordneten Anschlusskanal (4) und mindestens einem bezüglich der Längsachse (A) des Grundkörpers (2) exzentrisch angeordneten Durchflusskanal (7, 7')am Durchflusssteuerelement (6) herstellbar ist.

12. Injektionsspritze (1) nach Anspruch 11, **dadurch gekennzeichnet, dass** neben zumindest einem bezüglich der Längsachse (A) des Grundkörpers (2) exzentrisch angeordneten Durchflusskanal (7, 7') am Durchflusssteuerelement (6) ein weiterer Durchflusskanal (7") vorhanden ist, welcher unabhängig von der Steuerstellung des Durchflusssteuerelementes (6) mit dem Anschlusskanal (4) des Grundkörpers (2) verbunden ist.

13. Injektionsspritze (1) nach Anspruch 12, **dadurch gekennzeichnet, dass** der weitere Durchflusskanal (7"), welcher unabhängig von der Steuerstellung des Durchflusssteuerelementes (6) mit dem Anschlusskanal (4) des Grundkörpers (2) verbunden ist, koaxial mit der Längsachse (A) des Grundkörpers (2) angeordnet ist.

14. Injektionsspritze (1) nach Anspruch 10 in Verbindung mit den Ansprüchen 3 und 5, **dadurch gekennzeichnet, dass** über das Dichtungselement (10) eine indirekte Fluidverbindung zwischen einem bezüglich der Längsachse (A) des Grundkörpers (2) exzentrisch angeordneten Anschlusskanal (4) und einem bezüglich der Längsachse (A) des Grundkörpers (2) exzentrisch angeordneten Durchflusskanal (7, 7') am Durchflusssteuerelement (6) herstellbar ist.

15. Injektionsspritze (1) nach Anspruch 10 in Verbindung mit den Ansprüchen 3 und 6, **dadurch gekennzeichnet, dass** über das Dichtungselement (10) eine indirekte Fluidverbindung zwischen einem bezüglich der Längsachse (A) des Grundkörpers (2) exzentrisch angeordneten Anschlusskanal (4) und einem mit der Längsachse (A) des Grundkörpers (2) koaxial angeordneten Durchflusskanal (7") am Durchflusssteuerelement (6) herstellbar ist.

16. Injektionsspritze (1) nach einem der Ansprüche 1 bis 15, **dadurch gekennzeichnet, dass** das Durchflusssteuerelement (6) Kopplungsmittel (13) zum Ankoppeln der Injektionsspritze (1) an eine weitere Vorrichtung umfasst.

17. Injektionsspritze (1) nach Anspruch 16, **dadurch gekennzeichnet, dass** die weitere Vorrichtung eine Schutzkappe (16), insbesondere mit einem Aufnahmeraum (17) zur Aufnahme einer Kanüle (12), umfasst, welche einen Sitz (18) für die Kopplungsmittel (13) am Durchflusssteuerelement (6) aufweist, wobei die Schutzkappe (16) bevorzugt einen Anschlussstutzen (19) zum Anschluss einer Fluidquelle und einen Transferkanal (20) zum Transfer eines Fluids vom Anschlussstutzen (19) zu einer am Sitz (18) ausgebildeten Mündungsöffnung (21) aufweist, wobei die Mündungsöffnung (21) vorzugsweise derart ausgebildet und am Sitz (18) angeordnet ist, dass bei im Sitz (18) angeordneten Kopplungsmitteln des Durchflusssteuerelementes (6) eine, insbesondere fluiddichte, vorzugsweise für Keime undurchlässige, Fluidverbindung zwischen der Mündungsöffnung (21) am Sitz (18) und einem Durchflusskanal (7) des Durchflusssteuerelements (6) besteht.

## Claims

1. Injection syringe (1) for dispensing a fluid, in particular for medical use, including
a) a basic body (2) which has a longitudinal axis (A) and comprises a preferably hollow-cylindrical receiving space (3) for receiving the fluid and a connection channel (4) which communicates with the receiving space (3), wherein the connection channel (4) opens out at a valve face (5) of the basic body (2),
b) a flow control element (6) which is arranged so as to be movable on the basic body (2) and comprises one or several flow channels (7, 7', 7"), wherein the flow channels (7, 7', 7") open out at a valve face (8) of the flow control element (6),
wherein the connection channel (4) of the basic body (2) and the flow channels (7, 7', 7") of the flow control element (6) are directly or indirectly fluidically connected to one another or are movable into fluidic connection as a result of a relative movement of the basic body (2) and the flow control element (6), wherein a sealing element (10) is arranged between the valve face (5) of the basic body (2) and the valve face (8) of the flow control element (6), wherein a cannula (12), in particular an injection needle or an irrigation cannula, is arranged or is arrangeable in such a manner on the flow control element (6) that there is a fluid-tight fluid connection to a flow channel (7', 7"), wherein the cannula (12) is fixedly inserted into the flow channel (7', 7'') and is arranged, in particular, parallel to the longitudinal axis (A) of the basic body (2).

2. Injection syringe (1) according to Claim 1, **characterized in that** the connection channel (4) on the basic body (2) extends substantially parallel to the longitudinal axis thereof (A) of the basic body.

3. Injection syringe (1) according to either of Claims 1 and 2, **characterized in that** the flow control element (6) is arranged on the basic body (2) so as to be rotatable about a rotational axis which is substantially parallel to the longitudinal axis (A) of the basic body (2) and the flow control element (6) is movable as a result of rotation between several control positions.

4. Injection syringe (1) according to Claim 2 in conjunction with Claim 3, **characterized in that** the connection channel (4) on the basic body (2) is arranged eccentrically with reference to the longitudinal axis (A) of the basic body.

5. Injection syringe (1) according to Claim 2 in conjunction with Claim 3, **characterized in that** the connection channel (4) on the basic body (2) is arranged coaxially with the longitudinal axis (A) of the basic body.

6. Injection syringe (1) according to one of Claims 3 to 5, **characterized in that** at least one flow channel (7, 7') on the flow control element (6) is arranged eccentrically with reference to the longitudinal axis (A) of the basic body (2).

7. Injection syringe (1) according to one of Claims 3 to 6, **characterized in that** a flow channel (7") on the flow control element (6) is arranged coaxially with the longitudinal axis (A) of the basic body (2).

8. Injection syringe (1) according to Claim 3, **characterized in that** on its valve face (5), the basic body (2) comprises at least one element for preventing a relative rotation of basic body (2) and sealing element (6).

9. Injection syringe (1) according to Claim 8, **characterized in that** the element for preventing a relative rotation of basic body (2) and sealing element (10) is developed as a mouth connecting piece (11) of the connection channel (4).

10. Injection syringe (1) according to one of Claims 1 to 9, **characterized in that** an indirect fluid connection is producible via the sealing element (10) between the connection channel (4) of the basic body (2) and at least one flow channel (7, 7', 7") of the flow control element (6).

11. Injection syringe (1) according to Claim 10 in conjunction with Claims 4 and 5, **characterized in that** by means of the sealing element (10), an indirect fluid connection is producible on the flow control element (6) between a connection channel (4), which is arranged coaxially with the longitudinal axis (A) of the basic body (2), and at least one flow channel (7, 7') which is arranged eccentrically with reference to the longitudinal axis (A) of the basic body (2).

12. Injection syringe (1) according to Claim 11, **characterized in that** along with at least one flow channel (7, 7') arranged eccentrically with reference to the longitudinal axis (A) of the basic body (2) on the flow control element (6), there is present a further flow channel (7") which is connected to the connection channel (4) of the basic body (2) irrespective of the control position of the flow control element (6).

13. Injection syringe (1) according to Claim 12, **characterized in that** the further flow channel (7"), which is connected to the connection channel (4) of the basic body (2) irrespective of the control position of the flow control element (6), is arranged coaxially with the longitudinal axis (A) of the basic body (2).

14. Injection syringe (1) according to Claim 10 in conjunction with Claims 3 and 5, **characterized in that** by means of the sealing element (10), an indirect fluid connection is producible on the flow control element (6) between a connection channel (4), which is arranged eccentrically with reference to the longitudinal axis (A) of the basic body (2), and a flow channel (7, 7') which is arranged eccentrically with reference to the longitudinal axis (A) of the basic body (2).

15. Injection syringe (1) according to Claim 10 in conjunction with Claims 3 and 6, **characterized in that** by means of the sealing element (10), an indirect fluid connection is producible on the flow control element (6) between a connection channel (4), which is arranged eccentrically with reference to the longitudinal axis (A) of the basic body (2), and a flow channel (7") which is arranged coaxially with the longitudinal axis (A) of the basic body (2).

16. Injection syringe (1) according to one of Claims 1 to 15, **characterized in that** the flow control element (6) includes coupling means (13) for coupling the injection syringe (1) to a further device.

17. Injection syringe (1) according to Claim 16, **characterized in that** the further device includes a protective cap (16), in particular with a receiving space (17) for receiving a cannula (12), which protective cap comprises a seat (18) for the coupling means (13) on the flow control element (6), wherein the protective cap (16), in a preferred manner, comprises a connecting piece (19) for the connection of a fluid source and a transfer channel (20) for transferring a fluid from the connecting piece (19) to a mouth opening (21) which is realized on the seat (18), wherein the mouth opening (21) is preferably realized and arranged on the seat (18) in such a manner that, with coupling means of the flow control element (6) arranged in the seat (18), there is a(n), in particular fluid-tight, fluid connection, which is preferably impermeable to germs, between the mouth opening (21) on the seat (18) and a flow channel (7) of the flow control element (6).

## Revendications

1. Seringue d'injection (1) pour distribuer un fluide, en particulier pour application médicale, comprenant :
a) un corps de base (2) avec un axe longitudinal (A), qui présente un espace de réception (3) de préférence cylindrique creux pour recevoir le fluide et un canal de raccordement (4) en liaison avec l'espace de réception (3), le canal de raccordement (4) débouchant au niveau d'une surface de soupape (5) du corps de base (2),
b) un élément de commande de débit (6) qui est disposé de manière déplaçable au niveau du corps de base (2) et qui présente un ou plusieurs canaux de passage (7, 7', 7"), les canaux de passage (7, 7', 7") débouchant au niveau d'une surface de soupape (8) de l'élément de commande de débit (6),
le canal de raccordement (4) du corps de base (2) et les canaux de passage (7, 7', 7") de l'élément de commande de débit (6) étant directement ou indirectement en liaison fluidique les uns avec les autres ou pouvant être amenés en liaison fluidique par un mouvement relatif du corps de base (2) et de l'élément de commande de débit (6), un élément d'étanchéité (10) étant disposé entre la surface de soupape (5) du corps de base (2) et la surface de soupape (8) de l'élément de commande de débit (6), une canule (12), en particulier une aiguille d'injection ou une canule de rinçage, étant ou pouvant être disposée au niveau de l'élément de commande de débit (6) de telle sorte qu'il s'établisse une liaison fluidique étanche aux fluides avec un canal de passage (7', 7''), la canule (12) étant insérée fixement dans le canal de passage (7', 7") et étant notamment disposée parallèlement à l'axe longitudinal (A) du corps de base (2).

2. Seringue d'injection (1) selon la revendication 1, **caractérisée en ce que** le canal de raccordement (4) au niveau du corps de base (2) s'étend essentiellement parallèlement à son axe longitudinal (A) du corps de base.

3. Seringue d'injection (1) selon l'une quelconque des revendications 1 et 2, **caractérisée en ce que** l'élément de commande de débit (6) est disposé au niveau du corps de base (2) de manière rotative autour d'un axe de rotation essentiellement parallèle à l'axe longitudinal (A) du corps de base (2) et l'élément de commande de débit (6) peut être déplacé par rotation entre plusieurs positions de commande.

4. Seringue d'injection (1) selon la revendication 2 en association avec la revendication 3, **caractérisée en ce que** le canal de raccordement (4) est disposé au niveau du corps de base (2) de manière excentrique par rapport à l'axe longitudinal (A) du corps de base.

5. Seringue d'injection (1) selon la revendication 2 en association avec la revendication 3, **caractérisée en ce que** le canal de raccordement (4) est disposé au niveau du corps de base (2) coaxialement par rapport à l'axe longitudinal (A) du corps de base.

6. Seringue d'injection (1) selon l'une quelconque des revendications 3 à 5, **caractérisée en ce qu'**au moins un canal de passage (7, 7') est disposé au niveau de l'élément de commande de débit (6) de manière excentrique par rapport à l'axe longitudinal (A) du corps de base (2).

7. Seringue d'injection (1) selon l'une quelconque des revendications 3 à 6, **caractérisée en ce qu'**un canal de passage (7") est disposé au niveau de l'élément de commande de débit (6) coaxialement par rapport à l'axe longitudinal (A) du corps de base (2).

8. Seringue d'injection (1) selon la revendication 3, **caractérisée en ce que** le corps de base (2) présente sur sa surface de soupape (5) au moins un élément pour empêcher une rotation relative du corps de base (2) et de l'élément d'étanchéité (6) .

9. Seringue d'injection (1) selon la revendication 8, **caractérisée en ce que** l'élément pour empêcher une rotation relative du corps de base (2) et de l'élément d'étanchéité (10) est réalisé sous forme de tubulure d'embouchure (11) du canal de raccordement (4).

10. Seringue d'injection (1) selon l'une quelconque des revendications 1 à 9, **caractérisée en ce qu'**entre le canal de raccordement (4) du corps de base (2) et au moins un canal de passage (7, 7', 7") de l'élément de commande de débit (6) une liaison fluidique indirecte peut être établie par le biais de l'élément d'étanchéité (10).

11. Seringue d'injection (1) selon la revendication 10 en association avec les revendications 4 et 5, **caractérisée en ce qu'**une liaison fluidique indirecte entre un canal de raccordement (4) disposé coaxialement par rapport à l'axe longitudinal (A) du corps de base (2) et au moins un canal de passage (7, 7') disposé de manière excentrique par rapport à l'axe longitudinal (A) du corps de base (2) peut être établie au niveau de l'élément de commande de débit (6) par le biais de l'élément d'étanchéité (10).

12. Seringue d'injection (1) selon la revendication 11, **caractérisée en ce qu'**en plus d'au moins un canal de passage (7, 7') disposé de manière excentrique par rapport à l'axe longitudinal (A) du corps de base (2) au niveau de l'élément de commande de débit (6), il est prévu un canal de passage supplémentaire (7") qui est connecté au canal de raccordement (4) du corps de base (2) indépendamment de la position de commande de l'élément de commande de débit (6).

13. Seringue d'injection (1) selon la revendication 12, **caractérisée en ce que** le canal de passage supplémentaire (7''), qui est connecté au canal de raccordement (4) du corps de base (2) indépendamment de la position de commande de l'élément de commande de débit (6), est disposé coaxialement par rapport à l'axe longitudinal (A) du corps de base (2).

14. Seringue d'injection (1) selon la revendication 10 en association avec les revendications 3 et 5, **caractérisée en ce qu'**une liaison fluidique indirecte entre un canal de raccordement (4) disposé de manière excentrique par rapport à l'axe longitudinal (A) du corps de base (2) et un canal de passage (7, 7') disposé de manière excentrique par rapport à l'axe longitudinal (A) du corps de base (2) peut être établie au niveau de l'élément de commande de débit (6) par le biais de l'élément d'étanchéité (10).

15. Seringue d'injection (1) selon la revendication 10 en association avec les revendications 3 et 6, **caractérisée en ce qu'**une liaison fluidique indirecte entre un canal de raccordement (4) disposé de manière excentrique par rapport à l'axe longitudinal (A) du corps de base (2) et un canal de passage (7") disposé coaxialement par rapport à l'axe longitudinal (A) du corps de base (2) peut être établie au niveau de l'élément de commande de débit (6) par le biais de l'élément d'étanchéité (10) .

16. Seringue d'injection (1) selon l'une quelconque des revendications 1 à 15, **caractérisée en ce que** l'élément de commande de débit (6) comprend des moyens d'accouplement (13) pour accoupler la seringue d'injection (1) à un dispositif supplémentaire.

17. Seringue d'injection (1) selon la revendication 16, **caractérisée en ce que** le dispositif supplémentaire comprend un capuchon de protection (16), en particulier avec un espace de réception (17) pour recevoir une canule (12) qui présente un siège (18) pour les moyens d'accouplement (13) au niveau de l'élément de commande de débit (6), le capuchon de protection (16) présentant de préférence une tubulure de raccordement (19) pour le raccordement d'une source de fluide et un canal de transfert (20) pour le transfert d'un fluide de la tubulure de raccordement (19) à une ouverture d'embouchure (21) réalisée au niveau du siège (18), l'ouverture d'embouchure (21) étant de préférence réalisée et disposée au niveau du siège (18) de telle sorte que lorsque des moyens d'accouplement de l'élément de commande de débit (6) sont disposés dans le siège (18), une liaison fluidique notamment étanche aux fluides, de préférence imperméable aux germes, soit établie entre l'ouverture d'embouchure (21) au niveau du siège (18) et un canal de passage (7) de l'élément de commande de débit (6).
